# EUROPÄISCHE PATENTSCHRIFT

(11) **EP 0 955 377 B1**
(45) Veröffentlichungstag und Bekanntmachung des Hinweises auf die Patenterteilung: **10.12.2003**
(21) Anmeldenummer: 99108890.7
(22) Anmeldetag: 05.05.1999
(51) Int. Cl.: C12Q 1/37, G01N 33/543, G01N 33/573, C07K 16/40

(54) **Verfahren zum Nachweis einer bei Apoptose aktivierten Protease**
Method for the detection of apoptosis activated protease
Méthode pour la détection de protease activée par apoptose

(30) Priorität: 07.05.1998 DE 19820328
(43) Veröffentlichungstag der Anmeldung: 10.11.1999
(73) Patentinhaber: Roche Diagnostics GmbH, 68298 Mannheim (DE)
(72) Erfinder: Fertig, Georg Dr., 82377 Penzberg (DE)

(56) Entgegenhaltungen:
- EP-A- 0 528 525
- WO-A-96/33268
- NICHOLSON D W ET AL: "IDENTIFICATION AND INHIBITION OF THE ICE/CED-3 PROTEASE NECESSARY FOR MAMMALIAN APOPTOSIS" NATURE, Bd. 376, Nr. 6535, 6. Juli 1995 (1995-07-06), Seiten 37-43, XP002013934 ISSN: 0028-0836

## Beschreibung

Die Erfindung betrifft ein Verfahren zum Nachweis von Apoptose bei eukaryontischen Zellen bzw. Apoptose-spezifischer Protease sowie einen für dieses Nachweisverfahren geeigneten Reagenzkit.

Der sogenannte programmierte Zelltod ist von großer Bedeutung für die Entwicklung und Funktionsfähigkeit von Geweben, Organen und Organismen als Ganzes (J. Cohen, Advances in Immunology 50 (1991), 55-85) und wurde bei einer Reihe von Krankheiten, wie Autoimmun- oder ischämischen Schädigungen, verschiedenen Krebsarten, Alzheimer und anderen neurodegenerativen Phänomenen beobachtet (G.M. Cohen, Biochem. J. 326 (1997), 1-16). Die auf diese Weise abgestorbenen Zellen zeichnen sich im Zytoplasma insbesondere durch mit Histonen assoziierte DNA-Fragmente als Mono- und Oligonukleosome aus. Ein solches Erscheinungsbild wird darüber hinaus bei induziertem Zelltod beobachtet, wie er beispielsweise durch ionisierende Strahlen (Yamada et al., Int. J. Radiat. Biol. 53 ( 1988), 65) oder bestimmte monoklonale Antikörper, wie beispielsweise Anti-Fas (Yonehara et al., J. Exp. Med. 169 (1989), 1747-1756) oder Anti-APO-1 (Trauth et al., Science 245 (1989), 301-304) ausgelöst wird. Auch zytotoxische T-Zellen und Natural-Killer-Zellen bewirken einen solchen induzierten Zelltod (s. z.B. S. Curnow et al., Cancer Immol. Immunother, 36 (1993), 149-155). Sie bewirken jedoch darüber hinaus eine erhöhte Permeabilität der Plasmamembran, wie sie bei nekrotischen Phänomenen beobachtet wird (Krähenbühl et al., Immol. Today 12 (1991), 389-402). Die beschriebene Art des programmierten oder induzierten Zelltods wird als Apoptose bezeichnet. Apoptose ist charakterisiert durch bläschenförmige Ausstülpungen der Plasmamembran, Kondensation des Chromatins und Aktivierung einer endogenen Endonuklease. Im Gegensatz zur Nekrose handelt es sich bei der Apoptose also um einen aktiven Prozeß der eukaryontischen Zelle. Die bei der Apoptose aktivierte Calcium- und Magnesium-abhängige Endonuklease spaltet den DNA-Doppelstrang in den leicht zugänglichen Linkerregionen zwischen den Nukleosomen in Mono- und Oligonukleosome. Die DNA in den Nukleosomen ist hingegen mit bestimmten Core-Histonen eng assoziiert und daher vor der Spaltung durch die Endonuklease geschützt (Burgoyne et al., Biochem. J. 143 (1974), 67; Stach et al., J. Neurochem. 33 ( 1979), 257). Daher ist nach Extraktion der DNA und Auftrennung in Agarosegel die für apoptotische Zellen typische "DNA-Leiter" zu erkennen, ein Muster von DNA-Fragmenten mit einer Länge von ca. 180 Basenpaaren bzw. einem Vielfachen davon (Wyllie, Nature 284 (1980), 555-556; J. Cohen, Advances in Immunology 50 (1991), 55-85). Die Plasmamembran der Zellen bleibt in diesem frühen Stadium der Apoptose intakt. Es kommt so zur Anreicherung der Mono- und Oligonukleosomen sowie anderer hoch molekularer Komponenten im Zytoplasma der sterbenden Zelle (Duke et al., Lymphokine Research 5 ( 1986), 289-299).

Obwohl in den letzten Jahren eine Reihe von Molekülen identifiziert werden konnten, die bei der biochemischen Entstehung apoptotischer Zellen eine Rolle spielen, und auch die fundamentale Bedeutung der Apoptose für biologische Prozesse, die von der Embryogenese bis zur Entwicklung des Immunsystems reichen, in Fachkreisen mehr und mehr anerkannt ist, ist der Mechanismus für die Entstehung der Apoptosis noch weitestgehend ungeklärt (M. Tewari et al., Cell 81 (1995), 801-809). Es gibt jedoch jüngst Anhaltspunkte dafür, daß bestimmte Proteasen, die zur Familie der Cystein-Proteasen, den sogenannten "CASPASEN" gehören, eine Schlüsselrolle bei der Entstehung von Apoptose spielen (D.W. Nicholson und N.A. Thornberry, TIBS 22 ( 1997), 299-306). Insbesondere konnte gezeigt werden, daß das Zymogen Caspase 3 (Cysteinyl-Asparaginsäure-Protease, CPP-32, Apopain, Yama) bei der Entstehung von Apoptose in aktivierter Form vorliegt (D.W. Nicholson et al., Nature 376 (1995), 37-43).

Testverfahren zum Nachweis von Apoptose bzw. Apoptose-spezifischer Proteasen sind beschrieben und beruhen im wesentlichen darauf, daß ein entsprechendes Peptidsubstrat gespalten und eine dabei freigesetzte fluoreszierende oder colorimetrische Komponente detektiert wird (z.B. D.W. Nicholson et al., Nature 376 ( 1995), 37-43). Von Nachteil bei den bestehenden Testverfahren zum Nachweis Apoptose spezifischer Proteasen ist jedoch, daß durch diese Verfahren auch andere an der Entstehung von Apoptose beteiligte Proteasen analysiert werden, die ebenfalls das eingesetzte Peptidsubstrat spalten, und somit die geforderte Spezifität nicht gegeben ist.

Aufgabe der Erfindung war daher, ein einfaches und spezifisches Testverfahren zum Nachweis von Apoptose, insbesondere in der frühen Entstehungsphase von Apoptosis, zur Verfügung zu stellen.

Diese Aufgabe wird gelöst durch ein Verfahren zum Nachweis von Apoptose-aktivierten Cysteinyl-Asparaginsäure-Proteasen, welches dadurch gekennzeichnet ist, daß man
- ein die zu untersuchende Zellpopulation beinhaltendes Lysat ohne Vormarkierung der zu untersuchenden Zellpopulation mit einem Bindungspartner inkubiert, der eine bei Apoptose aktivierte Protease spezifisch bindet, jedoch das aktive Zentrum der Protease nicht blockiert, und es sich bei dem Bindungsparnter um einen Antikörper handelt, der gegen das Fragment der Aminosäuren 1 bis 219 der humanen Cysteinyl-Asparaginsäure-Protease (CPP-32) gerichtet ist,
- mit einem für die Protease spezifischen und einer detektierbaren Gruppe gekoppeltem Peptidsubstrat und
- einem Reaktionspuffer in Kontakt bringt und
- die aus dem Substrat freigesetzte detektierbare Gruppe bestimmt.

Der Bindungspartner kann bei dem erfindungsgemäßen Verfahren entweder vor Inkontaktbringen mit der Probe bzw. dem Lysat an eine Festphase, wie beispielsweise eine Mikrotiterplatte oder Latexpartikel, gebunden sein oder wird nach Inkubation mit dem Proteasesubstrat an eine entsprechende Festphase gekoppelt. Als bei Apoptose aktivierte Proteasen kommen erfindungsgemäß insbesondere Cysteinyl-Asparaginsäure-Proteasen wie beispielsweise Caspase 3 oder Caspase 8 in Betracht.

Es hat sich überraschenderweise gezeigt, daß mit dem erfindungsgemäßen Verfahren apoptotische Zellen, d.h. durch programmierten oder induzierten Zelltod abgestorbene Zellen bereits ein Meßsignal nach etwa 1,5 bis 2 Stunden Inkubation und bei Existenz von weniger als etwa 1000 apoptotischen Zellen ergeben, ohne daß eine Vormarkierung der zu untersuchenden Zellpopulation erforderlich ist. Dadurch können auch in vitro nicht proliferierende Zellen untersucht werden. Das erfindungsgemäße Verfahren erlaubt ferner in einfacher und schnell durchführbarer Weise die quantitative Bestimmung des Ausmaßes der Apoptose in der zu untersuchenden Probe (Zellpopulation) und ist darüber hinaus äußerst sensitiv.

Zur Freisetzung der bei Apoptose aktivierten Protease(n) aus dem Zytoplasma apoptotischer Zellen wird die zu untersuchende Zellpopulation mit einem geeigneten Lyse-Puffer inkubiert, der im wesentlichen ein anionisches oder nicht-ionisches Detergenz, wie beispielsweise Triton, Tween oder Nonidet P40, in einer Konzentration von etwa 0,1 bis 2,0% (w/v) und eine im pH-Bereich von etwa 5,5 bis 9,0, bevorzugt von etwa 7 bis 9 puffernde Substanz enthält. Beispielsweise sind hier Phosphat, Tris·HCl und Hepes geeignet. Die Konzentration des Puffers beträgt etwa 1 bis 200 mM, vorzugsweise ca. 10 bis 80 mM, besonders bevorzugt sind etwa 50 mM. Wahlweise kann dem Lyse-Puffer darüber hinaus eine Sulfitgruppen reduzierende Substanz, wie beispielsweise Dithiotreitol (DTT) oder Dithioerythrit in einer Konzentration von etwa 0,1 bis 100 µM, und/oder Kochsalz zur Gewährleistung physiologischer Salzkonzentrationen zugesetzt werden.

Die Inkubation mit dem Lyse-Puffer erfolgt über einen Zeitraum von etwa einer bis fünf Minuten bei Temperaturen um 0 bis 4°C. Unlösliche Zellbestandteile werden durch Zentrifugation abgetrennt. Das auf diese Weise erhaltene Lysat wird zu einem entsprechenden Bindungspartner gegeben, wobei es sich bevorzugt um einen monoklonalen oder polyklonalen Antikörper handelt. Besonders bevorzugt sind erfindunsgemäß gegen eine bei Apoptose aktivierte Cysteinyl-Asparaginsäure-Protease bzw. ein diesbezügliches immunologisch aktives Fragment gerichtete monoklonale Antikörper. Als besonders geeignet hat sich erwiesen, wenn Antikörper verwendet werden, die mittels eines etwa 25 kDa großen Proteinfragments der humanen CPP-32-(Aminosäuren 1 bis 219) als Immunogen und durch dreimalige Injektion bevorzugt im Abstand von ca. zehn Tagen, erhältlich sind (z.B. Klon 19). Die auf diese Weise gewonnenen Klone sind, gegebenenfalls nach chromatographischer Reinigung gemäß dem Fachmann bekannter Methoden, für das erfindungsgemäße Verfahren besonders geeignet. Alternativ können entsprechende Antikörper von einschlägigen Anbietern, wie beispielsweise der Firma Transduction Laboratories, Lexington, KY (USA) kommerziell erworben werden.

Bei dem Peptidsubstrat für die Protease gekoppelt mit einer detektierbaren Gruppe handelt es sich beispielsweise um ein mit einer chromogenen oder fluoreszierenden Gruppe gekoppeltes Peptid bestehend aus zwei bis etwa 20 Aminosäuren. Bevorzugt enthält das Peptidsubstrat weniger als zehn Aminosäuren, besonders bevorzugt handelt es sich dabei um Tri- bis Octapeptide. Für das erfindungsgemäße Verfahren können darüber hinaus cyclische Peptidsubstrate mit integrierter Spaltsequenz, wobei das gespaltene Peptid ein Enzym zu dessen aktiver Form komplettiert, verwendet werden. Bevorzugte Peptidsubstrate weisen insbesondere Peptidfragmente folgender Aminosäuresequenzen auf: Trp (Leu)-Glu-His-Asp, Asp-Glu-Val (His)-Asp oder eine der folgenden drei Sequenzen Val (Leu)-Glu-His (Thr)-Asp auf. Die Peptid-Substrate können ferner acetyliert sein und beinhalten, bevorzugt an einer endständigen, nicht-acetylierten Aminosäure, die detektierbare Gruppe. Als detektierbare, d.h. chromogene oder fluoreszierende Gruppen kommen bevorzugt Coumarin-, Nitroanilid- oder Naphtylamidderivate in Betracht. Insbesondere haben sich erfindungsgemäß folgende Substrate als geeignet erwiesen: Acetyl-Asp-Glu-Val-Asp-7-Amido-4-trifluormethyl-cumarin (Acetyl-asparaginyl-glutaminyl-valinyl-asparaginyl-7-amido-4-fluoromethyl-cumarin; Ac-DEVD-AFC), Acetyl-Asp-Glu-Val-Asp-7-Amido-4-methylcumarin (Acetyl-asparaginyl-glutaminyl-valinyl-asparaginyl-7-amido-4-methyl-cumarin; Ac-DEVD-AMC), (7-Methoxycumarin-4-yl)acetyl-Asp-Glu-Val-Asp-Ala-Pro-Lys(2,4-dinitrophenyl)OH (7-Methoxy-cumarin-4-yl)acetyl-asparaginyl-glutaminyl-valinyl-asparaginylalaninyl-prolinyl-lysinyl(2,4-dinitro)phenol; Mca), (4-(4-Dimethylaminophenylazo)benzoyl-Asp-Glu-Val-Asp-5-[(2-aminoethyl)amino]-naphtalen-1-sulfonsäure ((4-(4-Dimethylaminophenylazo)benzoyl-asparaginyl-glutaminyl-valinyl-asparaginyl-5[(2-aminoethyl)amino]-naphthalen-1-sulfonsäure), oder Rhodamin-110-DEVD-Derivate.

Die Reaktion zwischen der die Protease enthaltenen Probe, dem Bindungspartner bzw. Antikörper und dem Proteasesubstrat kann entweder vor oder nach Bindung des Bindungspartners bzw.

Antikörpers an die Festphase erfolgen. Für die Inkubation verwendete Pufferlösungen beinhalten im wesentlichen eine im pH-Bereich von ca. 5,5 bis 9,0 puffernde Substanz in einer Konzentration von 1 bis 200 mM sowie eine Sulfitgruppen reduzierende Substanz, wie DTT oder Dithioerythrit in einer Konzentration von etwa 0,1 bis 100 µM, bevorzugt von etwa 5 bis 60 µM, und gegebenenfalls EDTA und/oder EGTA in einer Konzentration von ca. 0,1 bis 1,0 mM. Ferner kann der Puffer durch physiologische Kochsalzlösung und/oder ein Magnesiumsalz, bevorzugt etwa 1 mM, enthalten. Eine bevorzugte Ausführungsform der Erfindung ist ferner, wenn der für die Lyse der Zellen verwendete Puffer und der Reaktionspuffer identisch sind und ca. 0,1 bis 1% (w/v) eines entsprechenden Detergenzes sowie etwa 5 bis 60 µM einer Sulfitgruppen reduzierenden Substanz enthält.

Abhängig von der Anzahl der apoptotischen Zellen in der jeweiligen Probe kann die Inkubation bei einer Temperatur von ca. 37°C sowie pH 7,8 in einem Zeitraum von etwa 30 Minuten bis zwölf Stunden erfolgen. In den meisten Fällen hat sich eine Reaktionsdauer von 1,5 bis 5 Stunden als ausreichend erwiesen.

Als Negativkontrolle wird wie im Beispiel 1 beschrieben verfahren oder jeweils eine Parallelkultur der zu untersuchenden Zellpopulation, in der keine Apoptose induziert wurde, verwendet. Das ohne Zell-Lysate gemessene Signal liefert den Leerwert für die Bestimmung und wird von den Meßwerten abgezogen. Als positiv für das Vorliegen einer Apoptose sind solche Meßwerte für die in der Reaktionsmischung detektierbaren Gruppen zu werten, die reproduzierbar mindestens um den Faktor 2 über dem Meßwert ohne Induktion liegen. Als Parallelkultur der zu untersuchenden Zellpopulation, in der keine Apoptose induziert wurde, wird dabei eine Kultur von Zellen des gleichen Zelltyps und der gleichen Herkunft verwendet, bei welcher aufgrund der Vorgeschichte der verwendeten Zellen sowie der Kultivierungsbedingungen davon ausgegangen werden kann, daß keine Apoptose induziert wurde. Für den Fall, daß eine in vitro induzierte Apoptose bestimmt werden soll, wird die zu untersuchende Zellpopulation in zwei Parallelansätze aufgeteilt und dann in Gegenwart bzw. Abwesenheit des Apoptose induzierenden Agens (z.B. Camptothecin) vorinkubiert.

In einer bevorzugten Ausführungsform des erfindungsgemäßen Verfahrens liegt der verwendete Bindungspartner biotinyliert vor, um diesen vor, oder nach der Inkubation, an eine mit Streptavidin beschichtete feste Phase zu binden.

Mit Hilfe des erfindungsgemäßen Verfahrens ist es somit in einfacher Weise möglich, sowohl die Zytotoxizität von Zellen oder Verbindungen, welche Apoptose induzieren, als auch durch Apoptose spezifisch induzierte Proteasen zu bestimmen.

Ein weiterer Gegenstand der Erfindung ist daher die Verwendung des erfindungsgemäßen Verfahrens zur Bestimmung der zytotoxischen Wirkung oder Aktivität von zytotoxischen Zellen, Natural-Killer-Zellen, ionisierenden Strahlen oder chemischen Substanzen wie z.B. monoklonalen Antikörpern, Hormonen (z.B. Gluccorticoide) oder Toxinen (z.B. Dioxine). Die zytotoxische Wirkung oder Aktivität ergibt sich dabei aus dem über das erfindungsgemäße Verfahren bestimmten Ausmaß der Apopotose in der als Zielzellen verwendeten Zellpopulation.

Ein weiterer Gegenstand der Erfindung ist ein Reagenzkit zum Nachweis apoptotischer Zellen oder einer bei Apoptose aktivierten Protease in einer biologischen Probe ohne Vormarkierung der zu untersuchenden Zellpopulation, enthaltend einen Bindungspartner, der eine bei Apoptose aktivierte Protease spezifisch bindet, ohne das aktive Zentrum der Protease zu blockieren, ein für die Protease spezifisches Substrat und einen geeigneten Reaktionspuffer. Bei dem Bindungspartner handelt es sich um einen Antikörper, der gegen das Fragment der Aminosäuren 1 bis 219 der humanen Cysteinyl-Asparaginsäure-Protease (CPP-32) gerichtet ist. Der Bindungspartner (im Beispiel Caspase 3) kann dabei an eine feste Phase gekoppelt vorliegen, oder erst nach Inkubation an eine solche gekoppelt werden. Bevorzugte Ausführungsformen sind, wenn es sich bei dem Bindungspartner um einen monoklonalen oder polyklonalen Antikörper, der gegen ein etwa 25 kDa großes Proteinfragment der humanen CPP-32 gerichtet ist, handelt und/oder ein Fluoreszenz-markiertes Peptidsubstrat, wie beispielsweise Acetyl-Asp-Glu-Val-Asp-7-Amido-4-trifluormethyl-coumarin, verwendet wird.

Darüber hinaus hat sich die Verwendung des erfindungsgemäßen Reagenzkits zum Auffinden von solchen Substanzen als geeignet erwiesen, die befähigt sind, im Zustand der Apoptose aktivierte Proteasen zu inhibieren. Gleichfalls lassen sich Substanzen auffinden, die eine verstärkende, d.h. induzierende Wirkung auf bei Apoptose aktivierten Proteasen haben. Entsprechend inhibierende bzw. induzierende Substanzen können als Wirkstoffe, wie beispielsweise Arzneimittel eingesetzt werden. Durch Substanzen, die im Zustand der Apoptose aktivierte Proteasen zu 90% und mehr inhibieren, läßt sich beispielsweise das Absterben apoptotischer Zellen aufhalten oder der weitere Untergang zumindest verzögern. Als entsprechende inhibierende Substanzen haben sich beispielsweise Peptidderivate wie Acetylasparaginyl-glutaminyl-valinylasparaginsäurealdehyd (Ac-DEVD-CHO) oder Benzyloxycarbonyl-Valinyl-Alaninyl-DL-Asparaginsäurefluoromethylketone (Z-VAD-fmk) als geeignet herausgestellt. Darüber hinaus können Substanzen, die befähigt sind, im Zustand der Apoptose aktivierte Proteasen zu induzieren, als wertvolle Werkzeuge für die Gewinnung spezifischer Proteasen dienen. Insbesondere haben sich hier solche Substanzen als geeignet erwiesen, die die Aktivität entsprechender Proteasen mindestens um das 1,5fache verstärken können.

Bei den im Zustand der Apoptose aktivierten Proteasen handelt es sich insbesondere um Cystein- bzw. Cysteinyl-Asparaginsäure-Proteasen.

Die folgenden Beispiele erläutern die Erfindung näher.

### Beispiel 1: Protease-Aktivierung/Camptothecin

Zur Induktion von Apoptose in einem zellulären System wurden humane Zellen (U937; humanes histiolytisches Lymphom eines Kaukasiers) mit Camptothecin (Toposiomerase I Inhibitor, Chemotherapeutikum) behandelt. Bei Inkubation von 5x10⁵ Zellen/ml mit 4 µg/ml Camptothecin (CAM) für unterschiedliche Zeitintervalle, ist ein Anstieg der Proteaseaktivität bereits nach 2 h zu erkennen. Für den Nachweis von durch Apoptose aktivierte Protease (Caspase 3) werden aus der behandelten Zellkultur ca. 10⁶ Zellen entnommen und in Lysepuffer (50 mM NaPO₄, 10 mM NaCl, 1 mM MgCl₂, 0.25% (w/v) Nonidet P40, 0,3 mM EDTA; pH 7.8) auf Eis lysiert. Das Lysat wird dann zur spezifischen Caspase 3-Analyse mit einem gegen Caspase 3 gerichteten Antikörper in Kontakt gebracht, der an eine Festphase gekoppelt ist und Caspase 3 aus dem Lysat bindet. Nach Entfernen des Überstandes (enthält alle unspezifischen Bestandteile bzw. Enzyme) wird ein Substrat (50 µM Ac-DEVD-AFC) zugegeben und dessen Caspase 3 vermittelter Substratumsatz durch die Entstehung des fluoreszierenden Coumarin-Spaltproduktes gemessen. Nach Abzug des Leerwertes (Eigenfluoreszenz des Substrates ohne Lysatzugabe) kann der kinetische Verlauf der Caspase 3-Aktivität graphisch dargestellt werden (Abbildung 1 ).

### Beispiel 2: Farb-/Fluoreszenz-Substrat ohne spezifischen Bindungspartner

Als Funktionstest für das verwendete zelluläre Modell (U937/CAM), wurde ein unspezifischer Caspase Assay (Clonetech) eingesetzt, der zwar befähigt ist, Lysate zu analysieren, jedoch kein spezifisches Capturing der Caspase 3 erlaubt. Das verwendete Substrat ist wie in Beispiel 1 Ac-DEVD-AFC, parallel dazu wurde ein colorimetrisches Substrat (Ac-DEVD-pNA) eingesetzt. Unspezifisch sind diese Ansätze deshalb, weil jedes DEVD-Substrat sowohl von Caspase 3 als auch von Caspase 1, Casp 2 und Casp 7 gespalten wird.
Die Auswertung zeigt, daß das Apoptose-Modell prinzipiell funktioniert und sowohl das colorimetrische als auch das Fluoreszenz-Substrat zeigen eine Spaltung bei Apoptose-Induktion.

### Beispiel 3: Antikörperauswahl

Es wurden verschiedene, Caspase 3 erkennende Antikörper an eine Festphase (MTP) fixiert und damit die Caspase 3 (aktiviertes und nicht-aktiviertes Enzym) aus dem Lysat gebunden. Nach Zugabe des Fluoreszenz-Substrates (50 µM AMC) zeigte sich jedoch lediglich bei Verwendung eines erfindungsgemäßen Antikörpers (z.B. Klon 19) ein positives Signal. Die drei anderen verwendeten Antikörper binden zwar Caspase 3, geben jedoch kein signifikantes Signal, d.h. Protease-Aktivität kann nicht ermittelt werden.

**Tabelle 1:**

| MAK<CPP-32> | Clone 19 | PAK | Ab-1 | Ab-2 |
|---|---|---|---|---|
| - CAM | 2,38 | 2,59 | 2,34 | 2,214 |
| +CAM | 6,65 | 2,88 | 2,31 | 2,344 |
| Faktor | 2,79 | 1,11 | 0,99 | 1,06 |

### Beispiel 4: Auswahl des optimalen Fluoreszenz-Substrates

Es wurden drei Fluoreszenz-Substrate getestet. Es handelt sich hierbei um Acetyl-Asp-Glu-Val-Asp-7-Amido-4-trifluoromethyl-coumarin (AFC), Acetyl-Asp-Glu-Val-Asp-7-amido-4-methylcoumarin (AMC) und (7-Methoxycoumarin-4-yl)acetyl-Asp-Glu-Val-Asp-Ala-Pro-Lys(2,4-dinotrophenyl)OH (Mca). Die drei Substrate wurden miteinander verglichen, wobei die Auswertekriterien i) Background ii) Signalhöhe und iii) Signal-Ratio aus +/- Induktion waren. Es zeigt sich, daß das AMC-Derivat zwar die höchsten Signale generiert, jedoch die Eigenfluoreszenz des Substrates ohne Lysatzugabe relativ hoch ist. Das Mca-Substrat hingegen liefert ein etwas besseres Ratio, dagegen jedoch niedrige Signalhöhen. Beim AFC-Substrat hingegen erhält man ein optimales Ratio bei sehr guter Signalausbeute.

### Beispiel 5: Optimierung des Inkubationspuffers

Um einen optimalen Substratpuffer zur Verfügung zu stellen, mit welchem man gleichzeitig das Zell-Lysat herstellen kann, wurden verschiedene Pufferzusammensetzungen ausgetestet. Die diesbezügliche Auswertung zeigt, daß die Signalhöhen extrem von der Präsenz von DTT abhängig sind. Des weiteren zeigte sich das optimale Verhältnis und Signalausbeute in der Pufferzusammensetzung 2 der getesteten Lösungen. In Einzelfällen kann auf EDTA und/oder EGTA verzichtet werden, bei Anwendung ohne DTT zeigt allerdings die Anwesenheit von EDTA bzw.
EGTA eindeutige Vorteile (EDTA und EGTA sind nur in Puffer 2 u. 3 vorhanden, wobei dies die einzigen Pufferkombinationen sind, die ein positives Signal ohne DTT generieren). Der pH ist bevorzugt neutral bis schwach alkalisch, wobei eine breite pH-Spanne im Alkalischen noch zu akzeptablen Ergebnissen führt.

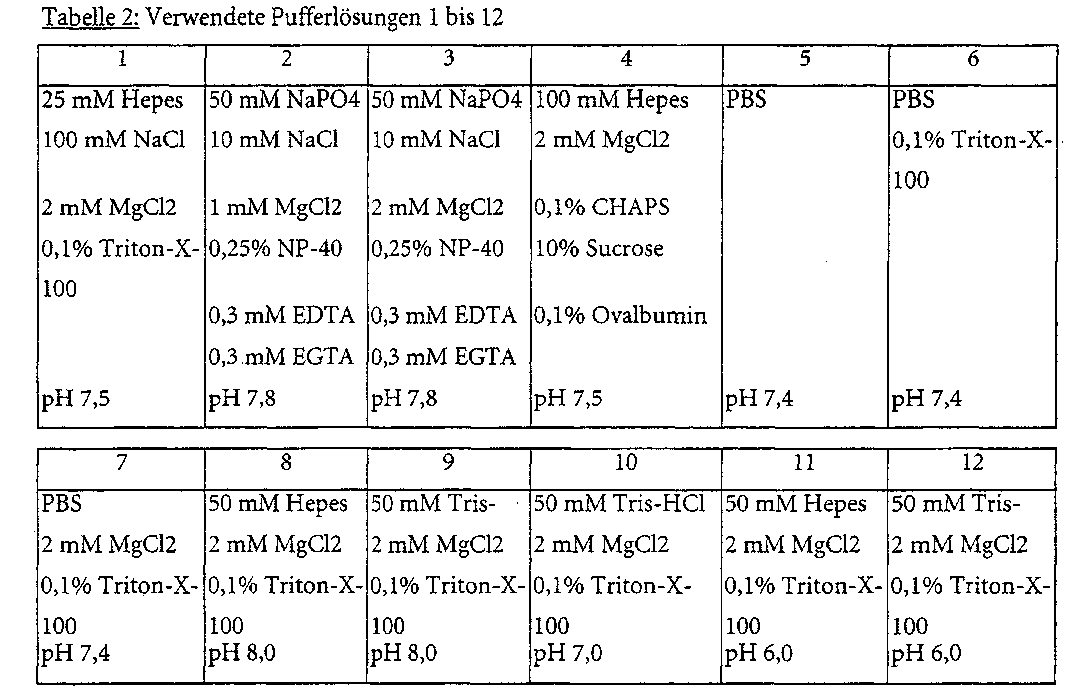

### Vorgehen:

MTP wurde mit 2µg/ml Anti-CPP32 (Clone 19) beschichtet (z.B. mit Carbonatpuffer)
Lysate von unbehandelten und Apoptose-induzierten Zellen aufpipettiert
Bindung von CPP32 (Caspase 3)
Waschen
Zugabe des Fluoreszenz-Substraten (AFC) im jeweiligen Inkubationspuffer
Messung der FU nach 2h

**Tabelle 3:**

| Pufferlösungen 1 bis 12 mit und ohne Zusatz von DTT | | | | | | | | | | | | |
|---|---|---|---|---|---|---|---|---|---|---|---|---|
| mit 10 µM DTT | | | | | | | | | | | | |
| Puffer | 1 | 2 | 3 | 4 | 5 | 6 | 7 | 8 | 9 | 10 | 11 | 12 |
| - CAM | 0,201 | 0,17 | 0,173 | 0,14 | 0,166 | 0,129 | 0,145 | 0,145 | 0,169 | 0,203 | 0,166 | 0,209 |
| + CAM | 2,349 | 2,907 | 2,499 | 1,715 | 2,059 | 2,174 | 2,234 | 0,969 | 1,656 | 1,492 | 0,494 | 0,994 |
| Faktor | 11,69 | 17,10 | 14,45 | 12,25 | 12,40 | 16,85 | 15,41 | 6,68 | 9,80 | 7,35 | 2,98 | 4,76 |

| ohne 10 µM DTT | | | | | | | | | | | | |
|---|---|---|---|---|---|---|---|---|---|---|---|---|
| Puffer | 1 | 2 | 3 | 4 | 5 | 6 | 7 | 8 | 9 | 10 | 11 | 12 |
| - CAM | 0,152 | 0,134 | 0,131 | 0,16 | 0,135 | 0,135 | 0,136 | 0,127 | 0,137 | 0,159 | 0,145 | 0,15 |
| + CAM | 0,185 | 0,337 | 0,334 | 0,145 | 0,12 | 0,015 | 0,125 | 0,121 | 0,113 | 0,184 | 0,14 | 0,181 |
| Faktor | 1,22 | 2,51 | 2,55 | 0,91 | 0,89 | 0,11 | 0,92 | 0,95 | 0,82 | 1,16 | 0,97 | 1,21 |

Ergebnis: 1) ohne DTT wird kaum ein Signal in Caspase 3 haltigem Lysat generiert
2) die Konzentration von DTT kann zwischen 1 und 100 µM liegen
3) Die Auswertung zeigt, daß die Pufferzusammensetzung 2 das höchste Signal (FU) und den größten Faktor (apopt. zu nicht-apopt. Lysat) produziert

### Beispiel 6: Antikörperherstellung

Zur Herstellung monoklonaler Antikörper gegen Caspase 3 wurde ein 24,7 kDa Protein Fragment (Aminosäuren 1-219 der humanen CPP-32) als Immunogen benutzt. Zur Immunisierung erhalten Mäuse (z.B. Balb/c) dreimal eine Injektion des Immunogens (ca. 50 µg Peptid) im Abstand von 10 Tagen. Nach etwa 50 Wochen (je nach Immunantwort des Serums) werden die Milzzellen der Serum-positiven Mäuse mit Myeloma Zellen (z.B. P3-X63-Ag8.653) fusioniert.
Die Hybridoma-Überstände werden auf positive Immunreaktion (Immunhistochemie, Westernblot) gescreent. Positive Hybridomas werden in "Limiting Dilution Verfahren" kloniert. Monoklonale Antikörper werden mittels chromatographischer Techniken aufgereinigt und in 20 mM Natriumphosphat pH 7,5, 50% Glycerin, 150 mM NaCl, 1 mg/ml RSA und 1,5 mM NaN₃ gelagert.

## Patentansprüche

1. Verfahren zum Nachweis einer bei Apoptose aktivierten Cysteinyl-Asparaginsäure-Protease oder apoptotischer Zellen in einer biologischen Probe ohne Vormarkierung der zu untersuchenden Zellpopulation, umfassend folgende Schritte:
- Inkontaktbringen der Probe mit (a) einem Bindungspartner, der eine bei Apoptose aktivierte Cysteinyl-Asparaginsäure-Protease spezifisch bindet, jedoch das aktive Zentrum nicht blockiert, es sich bei dem Bindungspartner um einen monoklonalen oder polyklonalen Antikörper handelt, der gegen das Fragment der Aminosäuren 1 bis 219 der humanen Cysteinyl-Asparaginsäure-Protease (CPP-32) gerichtet ist, und an eine Festphase gebunden ist bzw. bindbar ist, (b) einem für die Protease spezifischen Substrat und (c) einem Reaktionspuffer, und
- Bestimmung des in der Reaktionslösung entstandenen Chromogens oder Fluorochroms als Maß für die in der Probe enthaltenen apoptotischen Zellen und/oder aktivierte Protease.

2. Verfahren nach Anspruch 1, **dadurch gekennzeichnet, daß** die bei Apoptose aktivierte Protease eine humane Cysteinyl-Asparaginsäure-Protease ist.

3. Verfahren nach Anspruch 1, **dadurch gekennzeichnet, daß** der Antikörper mit einem 25 kDa-Fragment der humanen CPP-32 als Immunogen erhältlich ist.

4. Verfahren nach Anspruch 1, 2 oder 3, **dadurch gekennzeichnet, daß** ein Cumarin- oder Rhodaminpeptidderivat, para-Nitroanilid- oder ein Naphthylamidpeptidderivat als Proteasesubstrat verwendet wird.

5. Verfahren nach Anspruch 4, **dadurch gekennzeichnet, daß** es sich um Acetyl-asparaginyl-glutaminyl-valinyl-asparaginyl-7-amido-4-fluormethyl-cumarin bzw. -7-amido-4-methylcumarin, ein (7-Methoxy-cumarin-4-yl)acetyl-asparaginyl-glutaminyl-valinyl-asparaginyl-alaninyl-prolinyl-lysinyl(2,4-dinitro)phenolderivat oder 4-(4-Dimethylaminophenylazo)benzoyl-asparaginyl-glutaminyl-valinyl-asparaginyl-5-[(2-aminoethyl)amino]-naphthalen-1-sulfonsäure handelt.

6. Verfahren nach einem der Ansprüche 1-5, **dadurch gekennzeichnet, daß** der Reaktionspuffer einen pH-Wert von 5,5 bis 9,0 aufweist, ein Detergenz und eine Sulfitgruppen-reduzierende Substanz enthält.

7. Verfahren nach einem der Ansprüche 1-6, **dadurch gekennzeichnet, daß** in der Probe weniger als 1000 apoptotische Zellen vorliegen.

8. Verfahren nach einem der Ansprüche 1-7, **dadurch gekennzeichnet, daß** die Reaktion bei 37°C in einem Zeitraum von 30 Minuten bis zwölf Stunden durchgeführt wird.

9. Verfahren nach Anspruch 8, **dadurch gekennzeichnet, daß** die Reaktionsdauer 1,5 bis 5 Stunden beträgt.

10. Verwendung eines Verfahrens gemäß einem der Ansprüche 1 bis 9 zur Bestimmung der zytotoxischen Wirkung oder Aktivität von zytotoxischen Zellen, Natural-Killer-Zellen, ionisierender Strahlung oder chemischen Verbindungen wie insbesondere von Camptothecin, Dioxinen oder Antikörpern.

11. Reagenzkit zum Nachweis apoptotischer Zellen oder einer bei Apoptose aktivierten Cysteinyl-Asparaginsäure-Protease in einer biologischen Probe ohne Vormarkierung der zu untersuchenden Zellpopulation, enthaltend
(a) einen Bindungspartner, der eine bei Apoptose aktivierte Cysteinyl-Asparaginsäure-Protease spezifisch bindet, ohne das aktive Zentrum der Protease zu blockieren, und wobei es sich um einen Antikörper handelt, der gegen das Fragment der Aminosäuren 1 bis 219 der humanen CPP-32 gerichtet ist,
(b) ein für die Protease spezifisches Substrat, und
(c) Reaktionspuffer.

12. Reagenzkit nach Anspruch 11, **dadurch gekennzeichnet, daß** der Bindungspartner (a) an eine feste Phase gekoppelt vorliegt oder derart gestaltet ist, daß er an eine feste Phase koppelbar ist.

13. Reagenzkit nach Anspruch 11 oder 12, **dadurch gekennzeichnet, daß** es sich bei dem Bindungspartner um einen eine Cysteinyl-Asparaginsäure-Protease bindenden Antikörper handelt, der mit einem 25 kDa-Fragment der humanen CPP-32 als Immunogen erhältlich ist.

14. Reagenzkit nach einem der Ansprüche 11 bis 13, **dadurch gekennzeichnet, daß** es sich bei dem Protease spezifischen Substrat um Acetyl-asparaginyl-glutaminyl-valinyl-asparaginyl-7-Amido-4-trifluormethylcumarin handelt.

15. Verwendung des Reagenzkits gemäß einem der Ansprüche 11 bis 14 zum Auffinden einer Substanz, die befähigt ist, eine bei Apoptose aktivierte Protease zu inhibieren oder zu induzieren.

16. Verwendung nach Anspruch 15, **dadurch gekennzeichnet, daß** die Substanz die Protease zu mindestens 90% inhibiert.

17. Verwendung nach Anspruch 15, **dadurch gekennzeichnet, daß** die Substanz die Protease mindestens um das 1,5-fache induziert.

## Claims

1. Method for the detection of a cysteinyl-aspartic acid protease activated in apoptosis or of apoptotic cells in a biological sample without prelabelling the cell population to be examined comprising the following steps:
- contacting the sample with (a) a binding partner which specifically binds a cysteinyl-aspartic acid protease activated in apoptosis but does not block the active centre, the binding partner being a monoclonal or polyclonal antibody which is directed against the fragment of amino acids 1 to 219 of human cysteinyl-aspartic acid protease (CPP-32) and is bound or can be bound to a solid phase, (b) a substrate that is specific for the protease and (c) a reaction buffer and
- determining the chromogen or fluorochrome formed in the reaction solution as a measure for the apoptotic cells and/or activated protease contained in the sample.

2. Method as claimed in claim 1, wherein the protease activated in apoptosis is a human cysteinyl-aspartic acid protease.

3. Method as claimed in claim 1, wherein the antibody can be obtained using a 25 kDa fragment of human CPP-32 as the immunogen.

4. Method as claimed in claim 1, 2 or 3, wherein a coumarin-peptide derivative or rhodamine-peptide derivative, para-nitroanilide-peptide derivative or a naphthylamide-peptide derivative is used as the protease substrate.

5. Method as claimed in claim 4, wherein it is acetyl-asparaginyl-glutaminyl-valinyl-asparaginyl-7-amido-4-fluoromethyl-coumarin or -7-amido-4-methyl-coumarin, a (7-methoxy-coumarin-4-yl)acetyl-asparaginyl-glutaminyl-valinyl-asparaginyl-alaninyl-prolinyl-lysinyl(2,4-dinitro)phenol derivative or 4-(4-dimethylaminophenyl-azo)benzoyl-asparaginyl-glutaminyl-valinyl-asparaginyl-5-[(2-aminoethyl)amino]-naphthalene-1-sulphonic acid.

6. Method as claimed in one of the claims 1-5, wherein the reaction buffer has a pH value of 5.5 to 9.0 and contains a detergent and a substance which reduces sulphite groups.

7. Method as claimed in one of the claims 1-6, wherein less than 1000 apoptotic cells are present in the sample.

8. Method as claimed in one of the claims 1-7, wherein the reaction is carried out at 37°C for a period of 30 minutes to twelve hours.

9. Method as claimed in claim 8, wherein the reaction period is 1.5 to 5 hours.

10. Use of a method as claimed in one of the claims 1 to 9 to determine the cytotoxic effect or activity of cytotoxic cells, natural killer cells, ionizing radiation or chemical compounds such as in particular campthothecin, dioxins or antibodies.

11. Reagent kit for the detection of apoptotic cells or of a cysteinly-aspartic acid protease activated in apoptosis in a biological sample without prelabelling the cell population to be examined containing
(a) a binding partner which specifically binds a cysteinyl-aspartic acid protease activated in apoptosis without blocking the active centre of the protease, and where the binding partner is an antibody directed against the fragment comprising amino acids 1 to 219 of human CPP-32,
(b) a specific substrate for the protease and
(c) a reaction buffer.

12. Reagent kit as claimed in claim 11, wherein the binding partner (a) is present coupled to a solid phase or is designed such that it can be coupled to a solid phase.

13. Reagent kit as claimed in claim 11 or 12, wherein the binding partner is an antibody which binds a cysteinyl-aspartic acid protease and can be obtained using a 25 kDa fragment of human CPP-32 as an immunogen.

14. Reagent kit as claimed in one of the claims 11 to 13, wherein the protease-specific substrate is acetyl-asparaginyl-glutaminyl-valinyl-asparaginyl-7-amido-4-trifluoromethyl coumarin.

15. Use of the reagent kit as claimed in one of the claims 11 to 14 to find a substance which is able to inhibit or induce a protease activated in apoptosis.

16. Use as claimed in claim 15, wherein the substance inhibits the protease by at least 90 %.

17. Use as claimed in claim 15, wherein the substance induces the protease by at least 1.5-fold.

## Revendications

1. Procédé pour la détection d'une cystéinyle-acide aspartique protéase activée par apoptose ou de cellules apoptotiques au sein d'un échantillon biologique, sans marquage préalable de la population de cellules à analyser, comportant les étapes consistant à :
- mettre en contact l'échantillon avec (a) un partenaire de liaison, liant de manière spécifique une cystéinyle-acide aspartique protéase activée par apoptose, en ne bloquant toutefois pas le centre actif, le partenaire de liaison étant un anticorps monoclonal ou polyclonal, lequel est dirigé contre le fragment d'acides aminés 1 à 219 de la cystéinyle-acide aspartique protéase humaine (CPP-32) et qui est lié ou peut être lié à une phase solide, avec (b) un substrat spécifique à la protéase et (c) un tampon de réaction, et
- déterminer le chromogène ou le fluorochrome qui s'est produit dans la solution réactive, en tant que mesure pour les cellules apoptotiques et/ou la protéase activée contenu(s) dans l'échantillon.

2. Procédé selon la revendication 1,
**caractérisé en ce que** la protéase activée par apoptose est une cystéinyle-acide aspartique protéase humaine.

3. Procédé selon la revendication 1,
**caractérisé en ce que** l'anticorps peut être obtenu à l'aide d'un fragment de 25 kDa de la CPP-32 humaine, en tant qu'immunogène.

4. Procédé selon l'une quelconque des revendications 1, 2 ou 3,
**caractérisé en ce que**
un dérivé de peptide de coumarine ou un dérivé de peptide de rhodamine, un dérivé de peptide de paranitroanilide ou un dérivé de peptide de naphtylamide est utilisé en tant que substrat de protéase.

5. Procédé selon la revendication 4,
**caractérisé en ce qu'**il s'agit d'acétyl-asparaginyl-glutaminyl-valinyl-asparaginyl-7-amido-4-fluorométhyle-coumarine resp. de -7-amido-4-méthyle-coumarine, d'un dérivé (7-méthoxy-coumarine-4yl-) acétyl-asparaginyl-glutaminyl-valinyl-asparaginyl-alaninyl-prolinyl-lysinyl(2,4-dinitro)phénol ou de l'acide 4-(4-diméthylaminophényle-azo)benzoyl-asparaginyl-glutaminyl-valinyl-asparaginyl-5-[2-aminoéthyle)amino]- naphtalène-1-sulfonique.

6. Procédé selon l'une quelconque des revendications 1 à 5,
**caractérisé en ce que**
le tampon de réaction présente une valeur pH située entre 5,5 et 9,0 et contient un détergent et une substance réduisant les groupes de sulfites.

7. Procédé selon l'une quelconque des revendications 1 à 6,
**caractérisé en ce que**
le nombre de cellules apoptotiques présentes dans l'échantillon est inférieur à 1000.

8. Procédé selon l'une quelconque des revendications 1 à 7,
**caractérisé en ce que**
la réaction est réalisée à 37° C, au cours d'une période située entre 30 minutes et douze heures.

9. Procédé selon la revendication 8,
**caractérisé en ce que**
la durée de réaction est située entre 1,5 et 5 heures.

10. Utilisation d'un procédé selon l'une quelconque des revendications 1 à 9,
pour déterminer l'action cytotoxique ou l'activité de cellules cytotoxiques, de cellules tueuses naturelles, d'un rayonnement ionisant ou de composés chimiques, tels que en particulier la camptothécine, des dioxines ou des anticorps.

11. Ensemble de réactifs, pour la détection de cellules apoptotiques ou d'une cystéinyle-acide aspartique protéase activée par apoptose au sein d'un échantillon biologique, sans marquage préalable de la population de cellules à analyser, contenant
(a) un partenaire de liaison, liant de manière spécifique une cystéinyle-acide aspartique protéase activée par apoptose, en ne bloquant toutefois pas le centre actif de la protéase, le partenaire de liaison étant un anticorps, lequel est dirigé contre le fragment d'acides aminés 1 à 219 de la CPP-32 humaine,
(b) un substrat spécifique pour la protéase, et
(c) un tampon de réaction.

12. Ensemble de réactifs selon la revendication 11,
**caractérisé en ce que**,
le partenaire de liaison (a) est présent sous forme couplée à une phase solide ou est conçu de manière à pouvoir être couplé à une phase solide.

13. Ensemble de réactifs selon l'une quelconque des revendications 11 ou 12,
**caractérisé en ce que**,
le partenaire de liaison est un anticorps liant la cystéinyle-acide aspartique protéase, qui peut être obtenu à l'aide d'un fragment de 25 kDA de la CPP-32 humaine en tant qu'immunogène.

14. Ensemble de réactifs selon l'une quelconque des revendications 11 à 13,
**caractérisé en ce que**
le substrat spécifique à la protéase est de l'acétyl-asparaginyl-glutaminyl-valinyl-asparaginyl-7-amido-4-trifluorméthyl coumarine.

15. Utilisation de l'ensemble de réactifs selon l'une quelconque des revendications 11 à 14,
pour trouver une substance apte à inhiber ou à induire une protéase activée par apoptose.

16. Utilisation selon la revendication 15,
**caractérisée en ce que**
la substance inhibe la protéase à au moins 90 %.

17. Utilisation selon la revendication 15,
**caractérisée en ce que**
la substance induit la protéase d'au moins 1,5 fois.
